# EUROPEAN PATENT SPECIFICATION

(11) **EP 3 041 520 B1**
(45) Date of publication and mention of the grant of the patent: **27.04.2022**
(21) Application number: 14790002.1
(22) Date of filing: 03.09.2014
(51) Int. Cl.: A61L 15/22, A61F 13/15

(54) **DEVICE**
VORRICHTUNG
DISPOSITIF

(30) Priority: 03.09.2013 GB 201315649
(43) Date of publication of application: 13.07.2016
(73) Proprietor: Crawford Healthcare Ltd., Knutsford, Cheshire WA16 0BE (GB)
(72) Inventor: STEPHENSON, Christian, Wilmslow Cheshire SK9 2EP (GB)
(74) Representative: Simmons & Simmons
(86) International application number: PCT/EP2014/068737
(87) International publication number: WO 2015/032817

(56) References cited:
- EP-A1- 2 572 737
- US-A1- 2003 069 555
- US-A1- 2004 030 283
- US-A1- 2008 312 572
- US-B1- 6 632 974

## Description

The present invention relates to an absorbent and cushioning wound dressing and a method of manufacturing the wound dressing. The term 'absorbent and cushioning device' as used herein refers to medical absorbent and cushioning devices, such as wound dressings for chronic and burn wounds, and non-medical absorbent and cushioning devices, such as absorbent devices for the personal care sector, particularly for disposables, such as nappies (diapers) and incontinence products, and in particular refers to any such absorbent devices which also comprise a separate cushioning material, as defined herein.

The term 'cushioning wound dressing' as used herein refers to any absorbent wound dressing device which also comprises a separate cushioning material.

The term 'absorbent component' as used herein refers to any conformable component of an absorbent and cushioning device used for the absorption and retention in it of one or more bodily fluids from a body area, for example wound exudate from tissue in a wound. The absorbent component consists essentially of or comprises an absorbent material. The absorbent component is usually attached indirectly to a fluid-impermeable device backing layer.

The term 'absorbent material' as used herein refers to the absorbent material which is insoluble in, but absorbs a relevant bodily fluid and is essentially or is comprised in an absorbent component. Such a material may be in the form of a foam, fibres, an, often non-woven, fabric or a more random assembly of fibres, for example of randomly spun fibres, or in the form of a particulate.

The term "biocompatible" refers to any material which does not induce adverse effects such as immunological reactions and/or rejections in tissue in a human or animal.

The term "biodegradable" herein refers to any material which can be degraded and bioresorbed into, the physiological environment.

Examples include absorbent materials, for example cellulose and cellulose-based materials, such as carboxymethylcellulose materials.

The term "conformable container" herein in relation to any material in any component, for example an absorbent component, in an absorbent and cushioning device, for example a cushioning wound dressing, refers to any container, such as, depending on its contents, an optionally open-ended envelope, pouch or sachet, which contains the relevant material. The container comprises a proximal (body-facing) cover of a layer or sheet material that is substantially insoluble in but permeable to aqueous bodily fluid, for example wound fluid, and lies between the contained material and the relevant body area, for example wound tissue in use.

The term 'cushioning component' as used herein refers to any conformable component of an absorbent and cushioning device used for relieving pressure on a body area, for example tissue in and around a wound, from which an absorbent component in the device absorbs a bodily fluid, for example wound exudate. The cushioning component consists essentially of, or comprises a cushioning material. The cushioning component is usually attached directly or indirectly to a device backing layer.

The term 'cushioning material' as used herein refers to the cushioning material which is essentially or is comprised in a cushioning component. Such a material is insoluble in a relevant bodily fluid, and a soft, flexible, resiliently elastic material, and may be in the form of a porous material, such as a foam, fibres, an, often non-woven, fabric or a more random assembly of fibres, for example of randomly spun fibres, or in the form of a particulate.

The term "distal" herein in relation to a face of a component in a device means that in use the face faces away from the body in general, or away from the body in general, or away from a wound in a wound dressing.

The term "fluidic contact" in relation to a first component and a second component in an absorbent and cushioning device means that the first component is not directly in contact with the second component. The two components are separated by a third component which is fluid-permeable, including gas-permeable, such as moisture vapour transmitting.

Examples include an absorbent material and a cushioning material of an absorbent and cushioning device, where such a back layer of a stand-alone envelope between them holds them from direct contact.

The term "indirectly" in relation to the attachment of a first component of an absorbent and cushioning device to a second component means that the first component is attached directly to or comprised in a third component which is in turn attached directly to the second component.

The term "inner" herein in relation to any conformable container refers to any container which, together with its contents, is contained in another container, an "outer" container.

The term 'memory foam' as used herein with reference to the cushioning material which is essentially or is comprised in a cushioning component means a foam material, which is insoluble in a relevant bodily fluid, and is a soft, flexible, viscoelastic material with an open-cell solid structure. It is often a higher-density viscoelastic low-resilience hydrophilic polyurethane foam with additives which increase its viscosity and density, and matches pressure against it. In contact with a warm mammalian body, it softens in reaction to body heat, allowing it to mould to the body in a few minutes, yet slowly springs back to its original shape when the pressure is removed.

The term "non-biodegradable" herein refers to any material which cannot be degraded and bioresorbed into, the physiological environment. Examples include polyalkylenes, such as polyethylene, polypropylene and polybutylene, and combinations and copolymers thereof.

The term "non-woven" herein in relation to fabrics in the absorbent component means that the majority of fibres in the fabric are neither woven nor knit together, and are typically manufactured by putting small fibres together to form a sheet or web, and then binding them together by mechanical interlocking, with an adhesive, or thermally, in particular by spin bonding.

The term "outer" herein in relation to any conformable container refers to any container which, together with any other contents, contains another container, an "inner" container.

The term "proximal" herein in relation to a face of a component in a device means that in use the face is a body-facing face in general, or a wound-facing face in a wound dressing.

The term 'reticulated foam' as used herein with reference to the cushioning material which is essentially or is comprised in a cushioning component means a foam material, which is insoluble in a relevant bodily fluid, and is a soft, flexible, viscoelastic material with a structure with large open cells. It is often a hydrophilic polyurethane foam.

By "substantially water-insoluble" in relation to any material herein is meant that the material is soluble in an aqueous medium to less than 1% w/w.

The term "wound" herein means any soft tissue with compromised integrity, including acute wounds, such as surgical wounds infectious disease wounds; chronic wounds, such as diabetic ulcers or venous leg ulcers; and burns.

The term "wound contact integer" as used herein means a liquid-permeable component in a cushioning wound dressing, which comprises an absorbent component which is in direct or fluidic contact with, and a cushioning component.

The term "wound precursor" herein means any condition on or in the intact skin of a human or animal, or in the tissue underlying it, which in the absence of treatment is at substantial risk of developing into tissue of compromised integrity, such tissue including chronic wounds, including pressure sores, ulcers and infected wounds. Such conditions on or in the intact skin of a human or animal or in the tissue underlying it include discomfort, inflammation, pain and haematoma, which may be caused or exacerbated by pressure on the skin.

No statements made on belief herein, in particular as to the nature, physical form and properties of any integer or of any process shall be construed as limiting the present invention.

It is known to produce absorbent and cushioning devices for example a cushioning wound dressing, in which
a) the absorbent component and the cushioning component are the same integer, and
b) a material that is an absorbent material and a cushioning material, both as defined herein, is used to absorb one or more bodily fluids, for example wound exudate, and to relieve pressure on a body area, for example tissue around a wound.

The absorbent and cushioning component in such a device is usually attached directly or indirectly to a liquid-impermeable device backing layer on its distal side and a liquid-permeable body contact layer, for example a wound contact layer, on its proximal (wound-facing) side in use.

Examples of such wound dressings comprise polymeric materials as the absorbent and cushioning material, and include Allevyn Gentle ^{®} wound dressings used for the treatment of pressure-sensitive chronic wounds, such as diabetic ulcers or venous leg ulcers and Mepilex Border wound dressing ^{®} used for the treatment of a wide range of exuding wounds such as leg and foot ulcers, pressure ulcers, surgical incisions, and traumatic wounds, such as skin tears, blisters, abrasions and secondary healing wounds.

Each such conventional absorbent and cushioning device, for example a cushioning wound dressing, tend to have a perforated layer or sheet body-contact layer, for example a wound contact layer, with on its distal side an absorbent and cushioning component with a high absorbency usually attached directly or indirectly on its distal side in use to a liquid-impermeable device backing layer with a high moisture vapour transmission (MVT).

These properties of these integers have a positive effect on the degree and rate of absorption of bodily fluids, for example wound exudate, and its retention in the absorbent and cushioning component in such a device.

However, these positive effects in such an absorbent and cushioning device, for example a cushioning wound dressing, have a negative effect on the resilient elasticity of the material in the device, and it may eventually not be capable of providing adequate relief of pressure on the relevant body area, for example tissue around a wound.

Similarly, in a non-medical absorbent device, such as for the personal care sector, it may be desirable or necessary that
a) the device provide cushioning and optionally support to the area from which
b) the device is simultaneously absorbing bodily fluids, such as urine, to prevent pressure sores forming.

The same major disadvantage as for conventional cushioning wound dressings arises.

There is thus a need for an absorbent and cushioning device, in which a relatively high degree and rate of absorption and retention of bodily fluid from a bodily area (for example wound fluid) does not significantly impair the usual elastic resilience and dimensional stability of any cushioning material in the device such that its ability to provide optimum cushioning and optionally support to the area is impaired.

This could in general be achieved by having a cushioning component which lies away from, is separated from, and/or is not in direct and/or fluidic contact with the absorbent component. However, with conventional absorbent materials this tends to require multiple separate conformable containers, some with a liquid-permeable proximal face and a fluid-impermeable distal face, for example, one containing the absorbent material in contact with the cushioning component with concomitant increase in structural complexity and manufacturing cost.

It is also known to use such absorbent and cushioning devices for example a cushioning wound dressing to relieve pressure on a body area which is intact tissue around or comprising a wound precursor, as defined herein, to try to hinder or prevent it developing into tissue of compromised integrity, but with limited success.

Examples of such wound dressings include Allevyn Gentle ^{®} and Mepilex Border ^{®} wound dressings, normally used for the treatment of a wide range of exuding wounds, such as foot ulcers, pressure ulcers, surgical incisions, and traumatic wounds, such as skin tears, blisters, abrasions and secondary healing wounds.

There is thus a need for an absorbent and cushioning device to provide not only treatment of wounds by absorbing bodily fluids, such as exudate, but also to provide relief of pressure on pressure-sensitive areas of intact skin on the body that are particularly subject to pressure, and so provide treatment of wound precursors, as defined herein, and thus prophylaxis of wounds, such as pressure sores.

US 2008/312572 A1 concerns an absorbent article for application to human or animal skin surfaces. US 2003/069555 A1 concerns a padded absorbent article. US 6632974 B1 concerns an absorbent article. EP 2572737 A1 concerns a wound dressing. US 2004/030283 A1 concerns a disposable absorbent wound dressing with skin health treatment additives.

Accordingly, it is an object of the present invention to provide an absorbent and cushioning wound dressing which solves these problems. We have surprisingly found that all the above disadvantages may be overcome by an absorbent and cushioning wound dressing in which a cushioning component is in direct and/or fluidic contact with the distal face of an absorbent component, without any significant transfer in use to the cushioning component of any fluid absorbed into the absorbent material in the absorbent component, so that resilience of any cushioning material is not significantly impaired.

Accordingly, a first aspect of the present invention provides a conformable absorbent and cushioning wound dressing according to claim 1.

Preferably the absorbency of the absorbent material is 20 to 30 g/g of 0.9 % saline.

More preferably the absorbent material is a pharmaceutically acceptable salt of a poly(acrylic acid), of a poly(methacrylic acid) or a poly(acrylic-methacrylic acid) or a blend thereof.

In the present absorbent and cushioning wound dressing, when used to absorb a bodily fluid, the absorbency of the absorbent material enables large quantities of bodily fluid to be absorbed into the absorbent material in the device. The cushioning component is a separate integer from, but in direct or fluidic contact with the absorbent component. However, there is no transfer of the fluid from the distal face of the absorbent component to the cushioning component. The resilient elasticity of the material in the cushioning component is thus unimpaired, and it advantageously remains capable of providing adequate relief of pressure on the relevant body area, for example tissue around a wound.

The present absorbent and cushioning wound dressing may be used to relieve pressure on a body area which is dry intact tissue around or comprising a wound precursor, as defined herein, to try to hinder or prevent it developing into tissue of compromised integrity, such as a chronic wound; that is, to provide treatment of wound precursors, as defined herein, and thus prophylaxis of wounds, such as pressure sores.

The absorbent and cushioning wound dressing of the present invention surprisingly provide better prophylaxis than known absorbent and cushioning devices, such as the wound dressings Allevyn Gentle ^{®} and Mepilex Border ^{®}, it is believed, because the highly absorbent material in the absorbent component has a significant effect in regulating transpiration under the dressing and into the cushioning component, which is in direct or fluidic contact with the distal face of the absorbent component.

The structure, form and physical properties of the absorbent component, absorbent material and containers, including inner and outer containers, have a significant effect on the performance of the absorbent and cushioning device of the first aspect of the invention.

The cushioning component is mounted to be in direct or fluidic contact with, and at least partially overlie, the distal face of the absorbent component. The assembly of the absorbent component and the cushioning component may have a number of structures with different components of the dressing of different materials. All of these assemblies, however, comprise the distal face of the absorbent component at least partially covered by the proximal face of the cushioning component.

In a number of embodiments of a cushioning wound dressing of the first aspect of the invention:
a) the absorbent component may consist essentially of an absorbent material, or
b) the absorbent component may comprise the absorbent material, contained within a first container, which is a stand-alone envelope, and
c) the cushioning component may consist essentially of an elastically resilient cushioning material, or
d) the cushioning component may comprise the elastically resilient cushioning material, contained within a second container, which is a stand-alone envelope,

Each material is preferably present in the device as a layer and preferably a combination of b) with c) is contained in a third stand-alone envelope, and each stand-alone envelope has a fluid-permeable cover layer and back layer.

The absorbent material is usually attached indirectly to the proximal face of a backing layer of the device, by the fluid-permeable back layer of the third stand-alone envelope being attached directly via, for example, a layer of a pressure sensitive adhesive on the proximal face of the device backing layer, which in a dressing may be the same as that used for the purpose of securing it to the body of a patient, as described in further detail hereinafter.

Alternatively, however, it may be secured in a different manner, for example by a) or b) being attached directly to c) or d), and c) or d) being attached directly, again for example, by a layer of a pressure sensitive adhesive on the proximal face of the device backing layer. Again, the absorbent material is preferably in a first container which is a stand-alone envelope to form an absorbent component, and the cushioning material is preferably in a second container which is a stand-alone envelope.

There should be no appreciable gap between any mutually abutting faces of any of the components of the device. Such a structure should be such as to ensure that the cushioning component is in direct contact with the absorbent component.

As noted hereinbefore, it is an object of the present invention to provide an absorbent and cushioning wound dressing which solves the problems of the significantly impaired cushioning performance of known absorbent and cushioning devices, whilst at the same time avoiding the need for multiple separate conformable containers, some with a liquid-permeable proximal face and a fluid-impermeable distal face, for example, one containing the absorbent material in contact with the cushioning component, with concomitant increase in structural complexity and manufacturing cost.

It will be seen from the foregoing that the absorbent material may be used in or essentially as an absorbent component in a wide variety of forms in direct contact or fluidic contact through one or more conformable containers, with an elastically resilient cushioning material, and lower manufacturing cost. Any such containers will have a liquid-permeable proximal face and a fluid-permeable distal face.

It will be seen that the cushioning performance of the present absorbent and cushioning devices is not significantly impaired in use, whilst at the same time avoiding the need for multiple separate conformable containers, some with a liquid-permeable proximal face and a fluid-impermeable distal face, to prevent any significant transfer in use to the cushioning component of any fluid absorbed into the absorbent material in the absorbent component.

Embodiments of the dressing of the first aspect of the invention, and components thereof are described hereinafter in further detail.

The absorbent material in the absorbent and cushioning device, for example a cushioning wound dressing, may be in the form of a foam, fibres, an, often non-woven, fabric or a more random assembly of fibres, for example of randomly spun fibres, or preferably in the form of a particulate. The absorbent material may be contained in one or more conformable containers. The absorbent material in an absorbent and cushioning device, for example a cushioning wound dressing, may be a particulate or in any other form that is not attached indirectly to a device backing layer. It is then contained in a conformable container.

Where the material (or any other component of the device) in use might adhere to, the relevant body area, for example to wound tissue on removal of the absorbent and cushioning device, it is contained in such a container.

The absorbent and cushioning wound dressing, of the first aspect of the present invention is typically provided with a conformable device backing layer, to which the absorbent component is attached indirectly, through the cushioning component, as described in further detail hereinafter.

It is preferred that the absorbent material in the absorbent and cushioning device of the present invention, irrespective of its form, is present in the device as a layer in a conformable container. The layer may typically have a mean pre-absorption thickness of the order of 0.3 to 10, for example 0.7 to 5 mm. However, the properties of the absorbent material that prevent transfer of the fluid from the distal face of the absorbent component to the cushioning component appear to be independent of the thickness of this layer.

The container may be formed by a proximal (body-facing) fluid-permeable cover layer attached directly or indirectly to a device backing layer. Preferably, it will be a stand-alone envelope, with a fluid-permeable cover layer and a fluid-permeable back layer, which is optionally an inner container contained within an outer stand-alone envelope. The latter also has a fluid-permeable cover layer and a fluid-permeable back layer, which typically is bonded to a conformable backing layer, for example with a pressure sensitive adhesive.

As noted above, the absorbent material is optionally in the form of a foam, fibres, an, often non-woven, fabric or a more random assembly of fibres, for example of randomly spun fibres, or preferably in the form of a particulate.

Examples of suitable materials for an absorbent material include synthetic polymers, such as pharmaceutically acceptable salts of poly(acrylic and/or methacrylic) acids. Such materials are favourably cross-linked polyacrylate and polymethacrylate salts, for example alkali metal salts thereof, and copolymers and blends thereof. Preferred absorbent materials include cross-linked polyacrylate and polymethacrylate sodium salts, in particular cross-linked sodium polyacrylates, and blends thereof.

The absorbency of the absorbent material is in excess of 20, such as 20 to 30 g/g of 0.9 % saline.

Although this enables large quantities of bodily fluid (g/g of absorbent material in the device) to be absorbed into the absorbent material in the device, the nature and properties of this material prevent transfer of the fluid from the distal face of the absorbent component to the cushioning component. This in turn, in a non-medical device of the invention, such as for the personal care sector, prevents pressure sores forming, or in a medical device, such as a wound dressing, prevents pressure-sensitive chronic wounds, such as diabetic or venous leg ulcers, from worsening.

Similarly, when used to relieve pressure on a body area which is dry intact tissue around or comprising a wound precursor, as defined herein, provide treatment of wound precursors, as defined herein, and thus prophylaxis of wounds, such as pressure sores, the absorbent and cushioning wound dressing of the present invention surprisingly provide better prophylaxis than known absorbent and cushioning devices.

It is believed that this may be because the highly absorbent material in the absorbent component has a significant effect in regulating transpiration under the dressing and into the cushioning component.

In a first, preferred embodiment of the first aspect of the present invention there is provided a conformable absorbent and cushioning wound dressing that comprises a particulate form of the substantially water-insoluble absorbent material.

It will be appreciated that the absorbent material which is in the form of a particulate needs to be contained in a conformable container, preferably a stand-alone envelope. The stand-alone envelope is optionally an inner container within an outer container, which typically is bonded to a conformable backing layer, for example with a pressure sensitive adhesive, as described hereinabove in relation to the absorbent material in general.

The absorbent material is preferably present as a layer. Such a layer in a conformable container may typically have a mean pre-absorption thickness of the order of 0.3 to 10, for example 0.7 to 5 mm.

However, the properties of the absorbent material that prevent transfer of the fluid from the distal face of the absorbent component to the cushioning component appear to be independent of the thickness of this layer. The material may typically have a mean pre-absorption particle size of the order of 5 to 1000, for example 15 to 900 microns.

Suitable and preferred absorbent materials used in the absorbent component of this first embodiment of the absorbent and cushioning device of the present invention are as so described hereinabove in relation to the absorbent material.

In a second embodiment of the first aspect of the present invention there is provided a conformable absorbent and cushioning wound dressing, that comprises a, for example non-woven, fabric of fibres of one or more, preferably one, substantially water-insoluble, often hydrophilic absorbent materials.

The absorbent material may be in the form of at least one, preferably one, sheet, optionally bonded to each other. The total layer of a sheet, or optionally bonded sheets, may typically have a mean pre-absorption thickness of the order of 0.3 to 0, for example 0.7 to 5 mm. However, the properties of the absorbent material that prevent transfer of the fluid from the distal face of the absorbent component to the cushioning component appear to be independent of the thickness of this layer.

The absorbent material in the form of a, for example non-woven, fabric in the device according to the present invention may have a pre-absorption density of 32 to 128, and preferably 48 to 80 kg/m³ (2 to 8, and preferably 3 to 5, pounds/cubic/foot (pcf)).

The sheet, or optionally bonded sheets, may be attached directly to the proximal surface of the cushioning component, and optionally indirectly to a backing layer, through the cushioning component. Preferably, however, the sheet, or optionally bonded sheets, are contained in a conformable stand-alone envelope, which is optionally an inner container contained within an outer stand-alone envelope, also with a fluid-permeable cover layer and a fluid-permeable back layer, which typically is bonded to the conformable backing layer, for example with a pressure sensitive adhesive, as is described hereinabove in relation to the absorbent material in general.

In a third embodiment of the first aspect of the present invention, there is provided a conformable absorbent and cushioning wound dressing, that comprises a substantially water-insoluble, often hydrophilic absorbent material as a random assembly of fibres. The absorbent material may be in the form of at least one, preferably one, piece of wadding, such as a cushion or pad.

The pre-absorption fibres may have a monofilament linear density of 0.1 to 30, preferably about 0.5 to 20, and more preferably 0.9 to 4, for example 1 3 to 5 decitex, and a strength of 0.8 to 2.2, such as 1 to 2, for example 1.2 to 1.8 cN/dtex. It is preferred that the absorbent material in the form of a random assembly of fibres.

It is preferably a layer which may typically have a mean pre-absorption thickness of the order of 0.3 to 10, for example 0.7 to 5 mm.

However, the properties of the absorbent material that prevent transfer of the fluid from the distal face of the absorbent component to the cushioning component appear to be independent of the thickness of this layer.

At least one piece of wadding may be formed by spinning, optionally by electrospinning, or by melt-blowing. Spin-bonding of the component fibres is preferred.

Suitable and preferred absorbent materials used in the absorbent component of this first embodiment of the absorbent and cushioning device of the present invention are as so described hereinabove in relation to the absorbent material in general. The absorbent material is preferably one or more spun materials, such as a spun-bonded hydrophilic polymeric material.

The random assembly of fibres may be attached or spun, in particular with spin-bonding of the fibres, directly onto the proximal surface of the cushioning component. The latter may have at least one discontinuous area of an adhesive for the purpose of securing the random assembly of fibres. The cushioning component may be attached to the conformable backing layer, for example with a pressure sensitive adhesive.

Preferably, however, the random assembly of fibres is contained in a conformable stand-alone envelope, which is optionally an inner container contained within an outer stand-alone envelope, also with a fluid-permeable cover layer and a fluid-permeable back layer, which typically is bonded to the conformable backing layer, for example with a pressure sensitive adhesive, as is described hereinabove in relation to the absorbent material in general.

In a fourth embodiment of the first aspect of the present invention, there is provided an absorbent and cushioning wound dressing, which comprises a substantially water-insoluble, often hydrophilic absorbent material as a foam. It is preferred that the absorbent material is in the form of an open-cell foam.

The absorbent material is preferably a layer, which may typically have a mean pre-absorption thickness of the order of 0.3 to 10, for example 0.7 to 5 mm.

However, the properties of the absorbent material that prevent transfer of the fluid from the distal face of the absorbent component to the cushioning component appear to be independent of the thickness of this layer.

Suitable and preferred absorbent materials used in the absorbent component of this first embodiment of the absorbent and cushioning device of the present invention are as so described hereinabove in relation to the absorbent material in general.

The absorbent material may be in the form of at least one, preferably one, piece of open-cell foam, optionally bonded to each other. The piece of foam, or optionally bonded pieces, may be attached directly to the proximal surface of the cushioning component, and optionally indirectly to a backing layer, through the cushioning component being attached to the conformable backing layer, for example with a pressure sensitive adhesive.

Preferably, however, the piece of foam, or optionally bonded pieces, are contained in a conformable stand-alone envelope, which is optionally an inner container contained within an outer stand-alone envelope, also with a fluid-permeable cover layer and a fluid-permeable back layer.

The fluid-permeable back layer of the envelope or of the outer envelope as appropriate is typically bonded to the conformable backing layer, for example with a pressure sensitive adhesive, as is described hereinabove in relation to the absorbent material.

It will be seen from the foregoing that the absorbent material may be used in the present absorbent component in a wide variety of forms.

The function of the cushioning component in a wound dressing of the first aspect of the invention, is typically to provide relief of pressure on pressure-sensitive areas of the body that are particularly subject to pressure, whilst the absorbent component is simultaneously absorbing exudate at a relatively high degree and rate of absorption and retention from a chronic wound, for example a pressure sore, an ulcer, such as a diabetic foot ulcer or venous leg ulcer, or a burn wound. These potentially occur in patients, for example with arthritis, diabetes, cardiovascular conditions and/or paralysis.

The function of the cushioning component in a non-medical device (not according to the invention), such as for the personal care sector, particularly for disposable sanitary devices such incontinence products, is typically to provide relief of pressure on pressure-sensitive areas of intact skin on the body that are particularly subject to pressure, and so provide treatment of wound precursors, as defined herein, and thus prophylaxis of wounds, such as pressure sores, whilst simultaneously absorbing bodily fluids, such as urine,

The soles of the feet and the sacrum tend to be particularly pressure-sensitive areas of the body which may also require support. They are particularly subject to pressure and prone to developing sores, including pressure sores and/or ulcers and/or other forms of major inconvenience to patients referred to above. This tends to be particularly the case for the feet in patients with arthritis, diabetes or cardiovascular conditions who are not bedridden or chairbound. For such patients, and those with incontinence or paralysis, who are chairbound, the sacrum is particularly vulnerable.

In such cases, if such support of the area were required in the case of a medical device, it would commonly be provided by a separate integer, which will usually comprise a single mass of elastically resilient material shaped to conform in use to the relevant part of the body to provide support for that part of the body. This support may be made of one or more gas-permeable thermoplastic polymers or copolymers, for example a solid silicone, which comprises a silicone oil, a polyurethane or a polyester.

An absorbent and cushioning wound dressing of the first aspect of the invention preferably overlies, and preferably is essentially coterminous with, and is optionally attached directly to the proximal face of the shaped support.

The function of the cushioning component in a wound dressing of the first aspect of the invention is however typically to provide relief of pressure on pressure-sensitive areas, rather than to support the area. Accordingly, the conformable absorbent and cushioning device of the first aspect of the present invention is not often shaped to conform in use to the relevant part of the body.

The device is thus conveniently in a versatile lamellar shape such as an elongate strip, band or ribbon, a quadrilateral, such as a rectangle or oblong, or an equilateral, such as a square. This is referred to herein as an 'unshaped device'.

In the unshaped device, the absorbent component and cushioning component are also conveniently in a lamellar shape, referred to herein as an 'unshaped component'.

The proximal face of the cushioning component may be smaller than the distal face of the absorbent component. Preferably, however, the faces of the absorbent component and of the cushioning component are essentially coterminous. In both cases, however, as noted hereinbefore, there should be no appreciable gap between any mutually abutting faces of any of the components of the device, so that the cushioning component will only be in direct or fluidic contact with the absorbent material, and not with the relevant bodily fluid.

We have identified conventional cushioning components having an architecture, for example a foam, and being of materials, that make them elastically resilient, but which suffer loss of the initial elastic resilience if a bodily fluid, for example wound fluid, is able to infiltrate into and be retained by the cushioning material.

In combination with the absorbent material of the present invention they suffer no or negligible loss of the initial elastic resilience and also demonstrate good dimensional stability in the present absorbent and cushioning device, for example a cushioning wound dressing.

The cushioning component may comprise a cushioning material which is contained in its own conformable liquid-permeable container, optionally an inner container within an outer container. However, the cushioning component typically consist essentially of, a cushioning material.

As noted hereinbefore, the cushioning component on the distal face of the absorbent component is often an unshaped component in an unshaped device. The cushioning material is foam, in particular an open-cell foam.

Such materials tend to be substantially water-insoluble, elastically resilient synthetic polymeric materials including, but not limited to, hydrophilic polyurethanes, and copolymers and blends thereof.

Where the material of the cushioning component comprises a copolymer, examples of suitable copolymers include block copolymers of butylene with styrene.

The cushioning component is a substantially water-insoluble, elastically resilient hydrophilic polyurethane foam, which may include polyether polyurethane foams, typically the softest of grades with densities from 15 to 30 Kg/m³, and polyester polyurethane foams, typically with a more controlled cell structure with densities from 20 to 60 Kg/m³.

The cushioning component may additionally or alternatively comprise a microcellular foam material, often a microcellular polyurethane high density foams with good resistance to compression set, often a hydrophilic polyurethane foam, including a polyether polyurethane foam or a polyester polyurethane foam.

The cushioning component may additionally or alternatively comprise a reticulated foam as defined hereinbefore, that is, a foam material which is insoluble in a relevant bodily fluid, and is a soft, flexible, viscoelastic material with a structure with large open cells. It is often a hydrophilic polyurethane foam, including a polyether polyurethane foam, typically of 10 ppi to 60 ppi, or a polyester polyurethane foam, typically of 10 ppi to 90 ppi.

The cushioning component may additionally or alternatively comprise a memory foam as defined hereinbefore, that is, a foam material, which is insoluble in a relevant bodily fluid, and a soft, flexible, resiliently elastic material, often a higher density viscoelastic low-resilience polyurethane foam with additives which increase its viscosity and density and with an open-cell solid structure that matches pressure against it, and which in contact with a warm mammalian body preferably softens in reaction to body heat, allowing it to mould to the body in a few minutes, yet slowly springs back to its original shape when the pressure is removed.

Such a memory foam may be a gel memory foam, which consists of gel particles fused with memory foam to reduce body heat trapped under the dressing, speed up spring back time and help the dressing feel softer.

The cushioning component may comprise one or more, preferably one, layers of a substantially water-insoluble, elastically resilient polymeric material, optionally bonded to each other. The layer, or optionally bonded layers, may be attached directly to the proximal surface of a backing layer, for example with a pressure sensitive adhesive. The total layer may typically have a mean pre-absorption thickness of the order of less than 10 mm, for example 4 to 8, such as about 6 millimetres thick.

In a fifth, preferred embodiment, the cushioning component comprises a foam cushioning material, preferably one layer of a foam, in particular an open-cell foam.

The total layer may typically have a mean pre-absorption thickness of the order of less than 10 mm, for example 4 to 8, such as about 6 millimetres thick. The mean pre-absorption pore density may be for example from about 2 to 8, particularly from about 3 to 5 pcf.

Suitable and preferred materials used in the cushioning component of this fifth embodiment of the absorbent and cushioning device of the present invention are as so described hereinabove in relation to the cushioning material in general.

In a first form of this fifth embodiment of the absorbent and cushioning device of the present invention, the foam material used in the cushioning component is a single layer of foam. Suitable and preferred materials are as so described hereinabove in relation to such cushioning material in in that form.

In a second form of this fifth embodiment of the absorbent and cushioning device of the present invention, the cushioning component comprises a layer of a first foam material and a layer of a second, different foam material. Suitable and preferred materials are as described hereinbefore in relation respectively to such cushioning materials.

In this form of this embodiment, the two foam materials are usually present as two layers. The distal layer may be attached directly to the proximal surface of a backing layer, for example with a pressure sensitive adhesive.

The two foam layers may be bonded to each other and optionally both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, the two foam layers may not be bonded to each other. They may both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, when the two foam layers are not bonded to each other, one may be within a conformable (outer) outer container in the form of a stand-alone envelope, having a proximal cover layer and distal back layer, both permeable to bodily fluid. This may also contain the absorbent component in a conformable stand-alone inner container containing the absorbent material. The other foam layer will then lie distally of and outside the (outer) container. The total layer may typically have a mean pre-absorption thickness of the order of less than 10 mm, for example 4 to 8, such as about 6 millimetres thick.

The cushioning component may alternatively comprise a first foam, partly or wholly enclosing a second foam, for example to form a core within the latter within the device. Again the total layer may typically have a mean pre-absorption thickness of the order of less than 10 mm, for example 4 to 8, such as about 6 millimetres thick.

In a third form of this fifth embodiment of the absorbent and cushioning device of the present invention, the foam material used in the cushioning component is an open-cell memory foam as a single layer, and suitable and preferred materials are as so described hereinabove in relation to such cushioning material in in that form.

In a fourth form of this fifth embodiment of the absorbent and cushioning wound dressing of the present invention, the cushioning component comprises
a) an open-cell memory foam as defined hereinbefore, and
b) an open-cell non-memory foam.

Suitable and preferred materials are as described hereinbefore in relation respectively to such cushioning materials.

In this form of this embodiment, the two foam materials may be present as two layers, in either order distally.

The distal layer may be attached directly to the proximal surface of a backing layer, for example with a pressure sensitive adhesive. The two foam layers may be bonded to each other and optionally both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, the two foam layers may not be bonded to each other. They may both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, when the two foam layers are not bonded to each other, one may be within a conformable (outer) outer container in the form of a stand-alone envelope, having a proximal cover layer and distal back layer, both permeable to bodily fluid. This may also contain the absorbent component in a conformable stand-alone inner container containing the absorbent material. The other foam layer will then lie distally of and outside the (outer) container.

In this form, in the cushioning component, the memory foam will often lie distally of the non-memory foam. The total layer may typically have a mean pre-absorption thickness of the order of less than 10 mm, for example 4 to 8, such as about 6 millimetres thick.

The cushioning component may alternatively comprise a memory foam as defined hereinbefore, partly or wholly enclosed by a non-memory foam, for example to form a core within the latter within the device.

Less usually the memory foam may partly or wholly enclose the non-memory foam, for example for the latter to form a core within the former.

Again the total layer may typically have a mean pre-absorption thickness of the order of less than 10 mm, for example 4 to 8, such as about 6 millimetres thick.

In a fifth form of this fifth embodiment of the absorbent and cushioning wound dressing of the present invention, the cushioning component comprises
a) an open-cell foam, and distally thereof
b) a lamina of conformable, elastic material distally thereof.

Suitable and preferred foam materials are as described hereinbefore in relation respectively to such cushioning materials.

The lamina may be of a soft, pliable, almost viscous fluid-like plastics material, preferably of a conformable known gel or elastomer of this type. The gel material, for example, may consist of a silicone-based gel or another appropriate elastomer, for example one based on one or more alkylenes, such as ethylene, propylene and butylene, and styrene and combinations thereof, and thermoplastic polyurethanes, and certain copolyesters and polyamides, and combinations thereof.

Suitable silicone-based gel materials include cross-linked polydimethylsiloxanes which may optionally comprise up to 90 wt%, for example up to 60 wt%, such as up to 40 wt% of an oil, for example a silicone oil, for example a linear polydimethylsiloxanes. These gels have high durability and a useful level of tack, and are characteristically extremely soft and pliable, and can readily be shaped to conform in use to the body.

Suitable elastomers include for example one based on a combination of one or more alkylenes, such as ethylene, propylene and butylene, and styrene, in particular styrene ethylene butylene styrene (SBES), and thermoplastic polyurethanes. These are characteristically extremely soft and pliable.

Such plastics materials do not generally have good permeability to water vapour. It may thus be desirable to provide a plurality of channels running within the lamina between its proximal face and its opposite distal face and opening in a plurality of pores respectively on the proximal face and its opposing distal face. Mean density values of 1 to 15 per cm² and mean cross-sectional area values of 0.3 to 10 mm² may be suitable.

In this form of this embodiment, the distal lamina may be attached directly to the proximal surface of a backing layer, for example with a pressure sensitive adhesive.

The lamina and layer may be bonded to each other and optionally both be within a conformable (outer) container in the form of a stand-alone envelope, having a proximal cover layer permeable to bodily fluid. This may also contain the absorbent component in a conformable stand-alone inner container containing the absorbent material.

Alternatively, the lamina and layer may not be bonded to each other. They may both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, when the lamina and layer are not bonded to each other, the layer may be within a conformable (outer) container in the form of a stand-alone envelope, having a proximal cover layer and distal back layer, both permeable to bodily fluid. This may also contain the absorbent component in a conformable stand-alone inner container containing the absorbent material. The lamina will then lie distally of and outside the (outer) container.

The lamina and layer together may typically have a mean pre-absorption thickness of the order of less than 10 mm, for example 4 to 8, such as about 6 millimetres thick.

In a sixth form of this fifth embodiment of the absorbent and cushioning device of the present invention, the cushioning component consists essentially of a foam material (including a memory foam material) as described hereinbefore.

Suitable and preferred materials are as so described hereinabove in relation to such cushioning material in in that form. In all forms of this embodiment, preferably
a) a conformable outer container in the form of a stand-alone envelope, having a proximal cover layer and distal back layer, both permeable to bodily fluid, contains the assembly of
   i) the absorbent component in a conformable inner container containing the absorbent material in the form of a stand-alone envelope, having a proximal cover layer and distal back layer, both permeable to bodily fluid, and
   ii) the cushioning component,
b) the back layer of the outer container lies in direct contact with and is attached directly on its distal face to the proximal face of
c) a liquid-impermeable device backing layer with a high moisture vapour transmission (MVT), of at least 450g/m²/24hr.

Optionally a body contact layer in the form of a perforated layer overlies and extends beyond the proximal face of the absorbent component and is attached directly to the periphery of the proximal face of the backing layer.

In a sixth, less preferred embodiment, the cushioning component consists of at least one, preferably one, piece of wadding in the form of a random assembly of fibres of an elastically resilient polymeric material, preferably connected by being spun- bonded together.

The total layer may typically have a mean pre-absorption thickness of the order of less than 20 mm, for example 3 to 6 mm. Such flexible fibres typically have a monofilament linear density of 0.1 to 30, preferably about 0.5 to 20, and more preferably 0.9 to 4, for example 1 3 to 5 decitex, and a strength of 0.8 to 2.2, such as 1 to 2, for example 1.2 to 1.8 cN/dtex.

Suitable materials include optionally spun-bonded synthetic materials, such as non-biodegradable polymers such as spun-bonded polyalkylenes, for example polyethylenes, polypropylenes and polybutylenes, polyoxalates, polyamides, polycarbonates, polystyrene, and copolymers and blends thereof.

As noted hereinbefore, it is surprising advantage that, in use, when the device is applied to a body area, bodily fluids, such as wound exudate, will pass through any conformable container and infiltrate into the absorbent material of the device, but will not diffuse through to the cushioning component (whereby the elastic resilience of the cushioning material is unimpaired).

In a seventh, preferred embodiment of the first aspect of the present invention, any container is a stand-alone envelope, optionally as an outer container containing at least one inner container, which is a stand-alone envelope.

Any such container in any embodiment of the first aspect of the present invention has a fluid-permeable cover layer and a fluid-permeable back layer, so that the cushioning component is in fluidic contact with, the absorbent material. It is often convenient for the back layer to be made of the same fluid-permeable material and structure as the fluid-permeable cover layer.

Each layer may for example comprise a non-woven mesh of fibres characterised by having a monofilament linear density of 0.1 to 30, preferably about 0.5 to 20, and more preferably 0.9 to 4, for example 1 3 to 5 decitex, and a strength of 0.8 to 2.2, such as 1 to 2, for example 1.2 to 1.8 cN/dtex.

In any container, each layer may be of a hydrophobic polymeric material, which thus includes substantially water-insoluble materials, such as polymeric materials, for example polyalkylenes, such as polyethylene, polypropylene and polybutylene, polyoxalates, polyamides, polycarbonates and copolymers and blends thereof. Preferred materials include spun-bonded synthetic materials, such as polymers such as spun-bonded polyalkylenes, for example polyethylenes and polypropylenes and copolymers and blends thereof.

A distinct advantage of the absorbent and cushioning wound dressing, of the present invention, is that it does not require fluidic separation of the absorbent material and the cushioning component to retain the required elastic resilience and dimensional stability of the cushioning material to be retained. This would otherwise require, for example, at least one conformable container containing the absorbent material with a liquid-permeable proximal face and a fluid-impermeable distal face.

The wound dressing of the present invention which comprises a container liquid-permeable proximal face and a fluid-permeable distal face suffers no or negligible such loss of such initial elastic resilience and also demonstrate good dimensional stability.

The conformable absorbent and cushioning wound dressing of the first aspect of the present invention is provided with a backing layer which is preferably a gas-permeable barrier layer which is capable of forming a relatively liquid-tight seal on the body, but preferably has a high moisture vapour transmission (MVT).

Since there is no transfer in use to the cushioning component of any fluid absorbed into the absorbent material in the absorbent component, the backing layer is a perforated layer or sheet, for example to increase its moisture vapour transmission (MVT) over that of a continuous layer of a substantially insoluble polymeric material which is impermeable to the bodily fluid, for example wound exudate, without a concomitant increase in structural complexity and manufacturing cost.

Examples of suitable materials include substantially water-insoluble synthetic thermoplastic polymeric materials, such as polyalkylenes, for example polyethylenes, polypropylenes and polybutylenes, polyoxalates, polyamides, silicones, polyurethanes, polyesters and copolymers and blends thereof.

The backing layer will extend beyond the periphery of the distal face of the cushioning component, and will often bear at least one layer of pressure sensitive adhesive, for example a pressure sensitive acrylic adhesive.

In a medical device of the first aspect of the invention, which is a cushioning wound dressing, it will be on the proximal face of the device backing layer and used for the purpose of securing it to the body of a patient. In a non-medical device, such as for the personal care sector, particularly for disposable sanitary devices such as incontinence products, such a layer of pressure sensitive adhesive, for example a pressure sensitive acrylic adhesive will be on the distal face of the backing layer, and used for the purpose of securing it to the clothing of a user.

In both cases, the adhesive will extend beyond the periphery of the distal face of the cushioning component, and will often extend to the periphery of the relevant face of the backing layer. It may also have a high moisture vapour transmission (MVT).

In a medical device of the first aspect of the invention, this layer of pressure-sensitive, often acrylic, adhesive which is used for the purpose of securing the device onto a body area will preferably also be used for the purpose of securing the rest of the device to the proximal face of the backing layer. Any part of a layer of pressure-sensitive adhesive which is used for the purpose of securing the device onto a body area will preferably have release papers, such as release papers release-coated with polymers such as silicone, acrylate or polyurethane, which cover the layer of pressure-sensitive adhesive when it is not in use.

In an eighth embodiment of the dressing of the first aspect of the present invention, a body contact layer, for example a wound contact layer, overlies the absorbent component or an assembly of the absorbent component and the cushioning component. The body contact layer, for example is a perforated layer or sheet.

As noted hereinbefore, preferably, the faces of the absorbent component and of the cushioning component are essentially coterminous, and there should be no appreciable gap between any mutually abutting faces of any of the components of the device, so that the cushioning component will only be in direct or fluidic contact with the absorbent material, as described hereinbefore, and not in direct or fluidic contact with the relevant bodily fluid. For the same reason, the perforated part of the layer or sheet should not of course extend beyond the periphery of the absorbent component. The body contact layer is usually attached directly to a device backing layer.

In a medical device, such as for the treatment of pressure-sensitive chronic wounds, it may be attached directly to the proximal face of the backing layer by the layer of pressure-sensitive adhesive which is used for the purpose of securing the device onto a body area.

Alternatively, the perforated layer or sheet may be attached indirectly to the backing layer of the device.

I may, for example be attached directly on its distal face to the proximal face of the absorbent component or the assembly of the absorbent component and the cushioning component. The body contact layer may be attached by any liquid-permeable means, such as an adhesive in a discontinuous pattern across the face of the relevant integer, so that the absorbent component is in direct or fluidic contact with the relevant bodily fluid.

In a ninth embodiment of the first aspect of the present invention the conformable absorbent and cushioning device, for example a cushioning wound dressing, comprises a therapeutic agent.

Any such therapeutic agent should be pharmaceutically acceptable, and may suitably be any of the following pharmaceutically acceptable ingredients: antimicrobial agents, such as silver, silver oxides, iodine or chlorhexidine; agents that improve scar resolution and/or prevent scar formation, for example:
insulin, vitamin B, hyaluronic acid, mitomycin C;
growth factors (TGF[beta]);
cytokines;
corticosteroids,
agents that promote re-epithelialisation
topical analgesics, such as ibuprofen, diclofenac and capsaicin; to treat pain in combination with the soothing effect of the present cushioning device;
topical anaesthetics, such as lidocaine;
topical antiinflammatories, such as steroids, such as cortisol;
emollients, such as skin lipids and sterols, oils, for example lightweight oils, such as cetyl alcohol, or silicone-derived oils, such as cyclomethicone, and heavier oils, such as grape seed oil or dimethicone, and petrolatum;
antioxidants;
lubricants; and
soothing ingredients, such as chamomile and aloe vera;

The substance can be incorporated in the absorbent component and/or the absorbent material, before or during the manufacturing process for the absorbent and cushioning device. It may be preferred to incorporate it into the absorbent material of the device.

This provides convenience in manufacturing a device of the invention, since a therapeutic agent may be readily absorbed into the absorbent material, preferably in the form of a particulate, from an aqueous medium in which the absorbent material is substantially insoluble.

The skilled person will appreciate that, for regulatory reasons, the therapeutic agent and the absorbent material of the dressing should preferably bond to each other so strongly that leaching of the therapeutic agent from the absorbent material by a relevant bodily fluid that has passed through the proximal face of the absorbent component is hindered or prevented.

According to a tenth, preferred embodiment of the invention there is provided an absorbent and cushioning wound dressing 'the Preferred Dressing', with
a) an absorbent component which comprises a substantially water-insoluble particulate absorbent material, with a pre-absorption particle size of the order of 15 to 900 microns, in a layer with a mean pre-absorption thickness of the order of 0.7 to 5 mm, and of a cross-linked sodium polyacrylate, or a blend of cross-linked sodium polyacrylates
b) a conformable inner container containing the absorbent material in the form of a stand-alone envelope, having a proximal cover layer and distal back layer, both permeable to bodily fluid, and the back layer lying in direct contact with the proximal face of
c) a conformable cushioning component comprising at least one layer of an open-cell foam,
d) a conformable outer container in the form of a stand-alone envelope, having a proximal cover layer and distal back layer, both permeable to bodily fluid, and containing the assembly of the absorbent component and the cushioning component, the back layer lying in direct contact with and attached directly on its distal face to the proximal face of
e) a liquid-impermeable device backing layer with a high moisture vapour transmission (MVT), of at least 450g/m²/24hr,
f) optionally a body contact layer in the form of a perforated layer which overlies and extends beyond the proximal face of the absorbent component and is attached directly to the periphery of the proximal face of the backing layer.

Also disclosed is a method of manufacturing a conformable absorbent and cushioning wound dressing, of the first aspect of the invention.

The method comprises as a step a) at least partially covering the distal face of an absorbent component precursor with the proximal face of a cushioning component precursor.

In a first method, the method comprises as a step b) after step a) attaching the distal face of a cushioning component precursor directly or indirectly to the proximal face of a liquid-impermeable backing layer precursor.

In a preferred form of this method, the method comprises as a step b) after step a) attaching the distal face of a cushioning component precursor directly to the proximal face of a liquid-impermeable backing layer precursor.

In a preferred form of this method, the method comprises as a step c) before step b) enclosing the assembly of the absorbent component and the cushioning component precursors in a conformable outer container in the form of a standalone envelope, having a proximal cover layer and distal back layer, both permeable to bodily fluid, and attaching the back layer by its distal face directly to the proximal face of a liquid impermeable backing layer precursor.

In another preferred form of this method, the method comprises as a step d) before step a) enclosing a absorbent material precursor in a conformable inner container in the form of a stand-alone envelope, having a proximal cover layer and distal back layer, both permeable to bodily fluid, and attaching the back layer by its distal face directly to a liquid-impermeable backing layer precursor.

In another preferred form of this method, the method comprises as a step e) after all of steps a) to d) covering the proximal face of the absorbent component precursor with a perforated body contact layer which overlies and extends beyond the proximal face of the absorbent component precursor.

The absorbent component precursor optionally comprises a conformable inner container in the form of a stand-alone envelope. The perforated part of the body contact layer does not extend beyond the proximal face of the absorbent component precursor.

The perforated body contact layer is attached directly around its periphery to the periphery of the backing layer precursor.

Also disclosed, but not forming part of the invention, is a method of use of the device of the first aspect of the invention on a body area where relief of pressure and is desired or necessary, and from where bodily fluids are to be simultaneously absorbed, characterised by the application of a device of the first aspect of the invention to the body area.

In a first method, the method comprises a method of use of a non-medical device, such as for the personal care sector, particularly for disposable sanitary devices such incontinence products, characterised by the application of a nonmedical device of the first aspect of the invention to a body area of intact skin.

In a second, preferred method, the method comprises a method of treatment of pressure-sensitive exuding wounds, characterised by the application of a medical device of the first aspect of the invention to such a wound.

In this second, preferred method, the medical device may be a cushioning wound dressing, and the method comprises covering a pressure-sensitive exuding wound and the surrounding body area where relief of pressure and protection is desired with the medical device, and attaching it to the body area. The dressing can be easily applied to treat a wound, a chronic wound, for example a pressure sore, an ulcer, such as a diabetic foot ulcer or venous leg ulcer, or a burn wound.

In a third, preferred method, the method comprises a method of treatment of pressure-sensitive wound precursors and prophylaxis of wounds, characterised by the application of a medical device of the first aspect of the invention to a dry area of intact skin comprising the precursor.

In this third method, the medical device may be a cushioning wound dressing, and the method then comprises covering a dry area of intact skin comprising the precursor and the surrounding body area where relief of pressure and protection is desired with the medical device, and attaching it to the body area.

The dressing can be easily applied to treat a precursor of a wound, and thus to provide prophylaxis of a wound, for example a pressure sore, an ulcer, such as a diabetic foot ulcer or venous leg ulcer, or a burn wound.

The present invention is illustrated by the following Figure, which shows a cross-sectional side view of one form of a device of the first aspect of the invention.

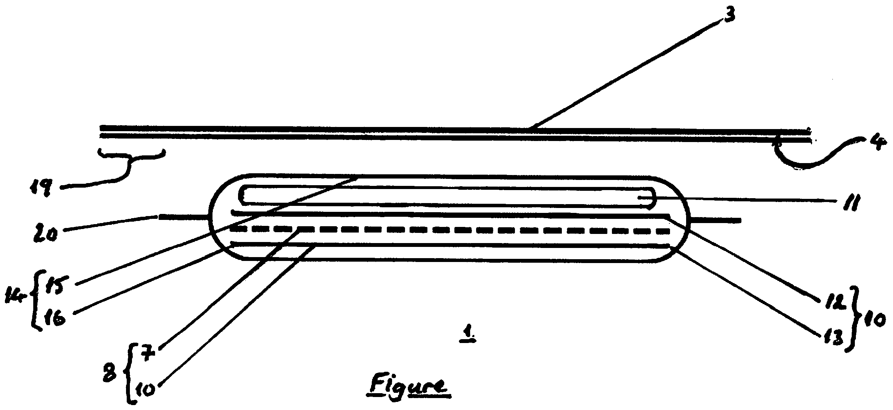

Referring to the Figure, a conformable absorbent and cushioning device, here a dressing 1, comprises an absorbent component 8 and a cushioning component 11, in which
a) the absorbent component 8 comprises a hydrophilic polymeric absorbent material 7 which is a pharmaceutically acceptable salt of a poly(acrylic acid), a poly(methacrylic acid) or a poly(acrylic-methacrylic acid) or a blend thereof, and
b) a cushioning component 11, which is a separate integer from, but in fluidic contact with, and at least partially overlies the distal face of, the absorbent component 7.

The absorbent component 8 comprises an absorbent material 7, here a particulate of a substantially water-insoluble hydrophilic material, here a sodium polyacrylate superabsorbent. The material 7 is contained in an inner container 10 in the form of a stand-alone envelope. The distal 12 and proximal 13 faces of the container 10 are of a water-insoluble, non-woven fabric, here of a blend of spun-bonded flexible fibres of a polyethylene - polyethylene terephthalate.

The cushioning component 11 consists essentially of a layer of an open-cell foam, here a soft-grade hydrophilic polyether polyurethane foam (alternatively a hydrophilic polyester polyurethane foam).

The absorbent component 8 compring the absorbent material 7 in the inner container 10 and the cushioning component 11 are in turn contained in an outer container 14, also in the form of a stand-alone envelope. The distal 15 and proximal 16 faces of the container 14 are made of the same material as that of the inner container 10.

The dressing 1 also comprises an elastically resilient gas-permeable, liquid-impermeable, relatively high-MVT film backing layer 3, here a polyurethane film layer.

The backing layer 3 overlies and extends beyond the periphery of the outer container 14 to form a border 19 on a part of the proximal face 4 of the backing layer 3, around the periphery 20 of the outer container 14.

A layer 17 of pressure-sensitive adhesive, here a pressure sensitive acrylic adhesive, extends across the proximal face 4 of the backing layer 3 and across the border 19, and is the means by which the proximal face 4 of the backing layer 3 is directly adhered to the absorbent component 6.

On the border 19 around the periphery 20 of the outer container 14, it is used for the purpose of securing the backing layer 3 of the dressing 1 over a wound, to form a relatively liquid-tight closure to exudate from the wound. It is covered with release papers (not shown) release-coated with a silicone, when the dressing 1 is not in use.

In use, the silicone-coated release papers are removed from the layer 17 of pressure-sensitive adhesive on the border 19 around the periphery 20 of the outer container 14, and the pressure sensitive acrylic adhesive is used to secure the conformable device backing layer 3 of the dressing 1 to the body of a patient across a wound, for example a pressure sore, or an ulcer, such as a diabetic foot ulcer.

Exudate absorption through respectively the proximal 16, 13 faces of the outer container 14 and of the inner container 10 and its absorption into and retention by the absorbent material 7 in the inner container 10 occurs. Although the cushioning component foam 11 is in direct contact with the absorbent component 8, when the latter is saturated the foam 11 remains dry, and the resilient elasticity of the material in the cushioning component 11 is unimpaired on exudate absorption and retention by the dressing. The foam therefore remains able to act as a cushion and dissipate pressure, giving constant pressure relief through wearing the dressing.

The present invention is also illustrated by the following comparison test Example:

### Example

Comparison of exudate absorption and retention of resilience of cushioning material by
a) a dressing A with a cushioning component as a separate integer from an absorbent component, and
b) a dressing B with a single integer cushioning and absorbent component.

The dressing A was the Preferred Dressing of the tenth embodiment of the first aspect of the invention, which comprises an absorbent component which comprises a substantially water-insoluble particulate of a cross-linked sodium polyacrylate. The particulate has a pre-absorption particle size of the order of 15 to 900 microns, in a layer with a mean pre-absorption thickness of the order of 0.7 to 5 mm, in an inner container.

The dressing B was Allevyn ^{®} (Smith & Nephew), used for the treatment of pressure-sensitive chronic wounds, with a resiliently elastic single cushioning component that is the same integer as the absorbent component.

An electronic pressure transference apparatus developed at the University of Bolton was used. The apparatus consists of a wooden platform for presentation of test specimens, a strain gauge device and an electronic circuit board.

A pressure pin (9mm diameter) is attached on to the load beam of the strain gauge and a corresponding hole drilled through the wooden platform. The height of the pressure pin is adjusted so that it protrudes through the hole of the platform by 1mm. This pin is used in order to simulate a bony prominence on a patient with low mobility.

Each specimen of A and B was tested dry (before the absorbent component absorbs and retains the fluid). Each specimen of A and B was then wetted, for 30 minutes at 37°C in Solution A, and then tested wet (after the absorbent component absorbed and retained the fluid). Each specimen of A and B, dry and wet, was placed onto the wooden platform over the pressure pin and a series of known metal block weights were placed onto its surface.

The strain gauge device detected the pressure transmitted through the specimen at each known pressure in increments created by the metal blocks. The amount of pressure absorbed and dissipated within the dressing structure and the actual pressure felt immediately below the specimen, for example a patient's leg, is determined. The transmitted pressure through the thickness of the specimen is the absolute pressure exerted on the patient's leg during treatment with the dressing.

The results are shown in the Table below:

**Force transmitted (g)**

| Mass Applied, g | The Preferred Dressing | The Preferred Dressing, wet | Allevyn | Allevyn, wet |
|---|---|---|---|---|
| 0 | 0 | 0 | 0 | 0 |
| 30 | 0 | 0 | 15 | 15 |
| 80 | 5 | 5 | 15 | 15 |
| 180 | 5 | 5 | 20 | 20 |
| 380 | 15 | 15 | 20 | 20 |
| 680 | 20 | 20 | 20 | 20 |
| 1180 | 35 | 35 | 25 | 25 |
| 2180 | 60 | 60 | 40 | 40 |
| 3180 | 85 | 85 | 85 | 100 |
| 4180 | 110 | 100 | 160 | 190 |
| 5180 | 130 | 105 | 255 | 280 |

### Conclusion

In the dressing A of the present invention, with a cushioning component that is a separate integer from, but in direct or fluidic contact with the absorbent component,
a) when the dressing is saturated the foam remains dry, and
b) the resilient elasticity of the material in the cushioning component is unimpaired on exudate absorption and retention by the dressing.

The foam therefore remains able to act as a cushion and dissipate pressure, giving constant pressure relief through wearing a dressing.

In the dressing B, Allevyn ^{®} (Smith & Nephew), with a cushioning component that is the same integer as the absorbent component, the resilient elasticity of the single cushioning and absorbent component is significantly impaired.

## Claims

1. A conformable absorbent and cushioning wound dressing which comprises an absorbent component, a cushioning component, and a backing layer, wherein
a) the absorbent component comprises a hydrophilic polymeric absorbent material which has an absorbency in excess of 20 g/g of 0.9 % saline;
b) the cushioning component comprises a substantially water-insoluble, elastically resilient hydrophilic polyurethane foam and which is a separate integer from, but in direct or fluidic contact with, and at least partially overlies the distal face of the absorbent component, wherein the distal face of the absorbent component faces away from the wound in use; and
c) the backing layer is a perforated layer or sheet.

2. A wound dressing according to claim 1 in which the absorbency of the absorbent material is 20 to 30 g/g of 0.9 % saline.

3. A wound dressing according to claim 1 in which the absorbent material
a) is a pharmaceutically acceptable salt of a poly(acrylic acid), of a poly(methacrylic acid) or a poly(acrylic-methacrylic acid) or a blend thereof,
b) comprises a particulate form of the substantially water-insoluble absorbent material,
c) comprises a non-woven fabric of fibres of one or more substantially water-insoluble, hydrophilic absorbent materials,
or
d) comprises a substantially water-insoluble, hydrophilic absorbent material as a random assembly of fibres.

4. A wound dressing according to claim 1 in which the hydrophilic polyurethane foam
a) is a hydrophilic polyether polyurethane foam or a polyester polyurethane foam
b) is a reticulated foam, or
c) is a viscoelastic low-resilience polyurethane memory foam.

5. A wound dressing according to claim 1 in which the foam is a gel memory foam.

6. A wound dressing according to claim 1 in which the cushioning component further comprises a lamina of a silicone-based gel or elastomer distally of the hydrophilic polyurethane foam.

7. A wound dressing according to claim 1 in which the absorbent component and the cushioning component are contained in a stand-alone envelope, as an inner container contained in an outer container, which is a stand-alone envelope.

8. A wound dressing according to claim 1 in which a perforated layer or sheet body contact
layer overlies the absorbent component or an assembly of the absorbent component and the cushioning component.

9. A wound dressing according to claim 1 which comprises a therapeutic agent.

10. A wound dressing according to claim 1 wherein the cushioning component is in direct contact with the absorbent component.

11. A method of manufacturing a wound dressing according to claim 1, which method comprises as a first step at least partially covering the distal face of an absorbent component precursor with the proximal face of a cushioning component precursor.

## Patentansprüche

1. Anpassbarer absorbierender und polsternder Wundverband, der eine absorbierende Komponente, eine polsternde Komponente und eine Trägerschicht umfasst, wobei
a) die absorbierende Komponente ein hydrophiles polymeres absorbierendes Material umfasst, das ein Absorptionsvermögen von mehr als 20 g/g von 0,9 % Kochsalzlösung aufweist;
b) die polsternde Komponente einen im Wesentlichen wasserunlöslichen, elastisch nachgiebigen hydrophilen Polyurethanschaum umfasst und der eine separate ganze Zahl ist, aber in direktem oder fluidischem Kontakt mit oder mindestens teilweise die distale Fläche der absorbierenden Komponente überdeckt, wobei die distale Fläche der absorbierenden Komponente im Gebrauch von der Wunde weg weist; und
c) die Trägerschicht eine perforierte Schicht oder Bahn ist.

2. Wundverband nach Anspruch 1, wobei das Absorptionsvermögen des absorbierenden Materials 20 bis 30 g/g von 0,9 % Kochsalzlösung beträgt.

3. Wundverband nach Anspruch 1, wobei das absorbierende Material
a) ein pharmazeutisch verträgliches Salz einer Poly(acrylsäure), einer Poly(methacrylsäure) oder einer Poly(acryl-methacrylsäure) oder einer Mischung davon ist,
b) eine Partikelform des im Wesentlichen wasserunlöslichen absorbierenden Materials umfasst,
c) ein Faservlies aus einem oder mehreren im Wesentlichen wasserunlöslichen, hydrophilen absorbierenden Materialien umfasst,
oder
d) ein im Wesentlichen wasserunlösliches, hydrophiles absorbierendes Material als eine zufällige Anordnung von Fasern umfasst.

4. Wundverband nach Anspruch 1, wobei der hydrophile Polyurethanschaum
a) ein hydrophiler Polyether-Polyurethanschaum oder ein Polyester-Polyurethanschaum ist
b) ein retikulierter Schaum ist oder
c) ein viskoelastischer Polyurethan-Gedächtnis-Schaum mit geringer Nachgiebigkeit ist.

5. Wundverband nach Anspruch 1, wobei der Schaum ein Gel-Gedächtnis-Schaum ist.

6. Wundverband nach Anspruch 1, wobei die polsternde Komponente weiter eine Schichtung aus einem Gel oder Elastomer auf Silikonbasis distal des hydrophilen Polyurethanschaums umfasst.

7. Wundverband nach Anspruch 1, wobei die absorbierende Komponente und die polsternde Komponente in einer eigenständigen Hülle enthalten sind, als ein innerer Behälter, der in einem äußeren Behälter enthalten ist, der eine eigenständige Hülle ist.

8. Wundverband nach Anspruch 1, wobei eine perforierte Schicht oder Bahnkörper-Kontaktschicht über der absorbierenden Komponente oder einer Anordnung aus der absorbierenden Komponente und der polsternden Komponente liegt.

9. Wundverband nach Anspruch 1, der einen therapeutischen Wirkstoff umfasst.

10. Wundverband nach Anspruch 1, wobei die polsternde Komponente in direktem Kontakt mit der absorbierenden Komponente ist.

11. Verfahren zur Herstellung eines Wundverbandes nach Anspruch 1, wobei das Verfahren als ersten Schritt das mindestens teilweise Bedecken der distalen Fläche eines Vorläufers einer absorbierenden Komponente mit der proximalen Fläche eines Vorläufers einer polsternden Komponente umfasst.

## Revendications

1. Pansement absorbant et de rembourrage adaptable qui comprend un composant absorbant, un composant de rembourrage et une couche de support, dans lequel
a) le composant absorbant comprend un matériau absorbant polymère hydrophile qui présente un pouvoir absorbant supérieur à 20 g/g de solution saline à 0,9 % ;
b) le composant de rembourrage comprend une mousse de polyuréthane hydrophile élastiquement souple, sensiblement insoluble dans l'eau et qui est une entité séparée de, mais en contact direct ou fluidique avec, et recouvre au moins partiellement la face distale du composant absorbant, dans lequel la face distale du composant absorbant est opposée à la plaie lors de son utilisation ; et
c) la couche de support est une couche ou une feuille perforée.

2. Pansement selon la revendication 1 dans lequel le pouvoir absorbant du matériau absorbant est de 20 à 30 g/g de solution saline à 0,9 %.

3. Pansement selon la revendication 1 dans lequel le matériau absorbant
a) est un sel pharmaceutiquement acceptable d'un poly(acide acrylique), d'un poly(acide méthacrylique) ou d'un poly(acide acrylique-méthacrylique) ou d'un mélange de ceux-ci,
b) comprend une forme particulaire du matériau absorbant sensiblement insoluble dans l'eau,
c) comprend un tissu non tissé de fibres d'un ou plusieurs matériaux absorbants hydrophiles, sensiblement insolubles dans l'eau,
ou
d) comprend un matériau absorbant hydrophile, sensiblement insoluble dans l'eau sous la forme d'un ensemble aléatoire de fibres.

4. Pansement selon la revendication 1 dans lequel la mousse de polyuréthane hydrophile
a) est une mousse de polyéther polyuréthane hydrophile ou une mousse de polyester polyuréthane
b) est une mousse réticulée, ou
c) est une mousse mémoire de polyuréthane de faible résilience viscoélastique.

5. Pansement selon la revendication 1 dans lequel la mousse est une mousse mémoire gel.

6. Pansement selon la revendication 1 dans lequel le composant de rembourrage comprend en outre une lamelle d'un gel ou d'un élastomère à base de silicone distale par rapport à la mousse de polyuréthane hydrophile.

7. Pansement selon la revendication 1 dans lequel le composant absorbant et le composant de rembourrage sont contenus dans une enveloppe autonome, en tant que contenant intérieur contenu dans un contenant extérieur, qui est une enveloppe autonome.

8. Pansement selon la revendication 1 dans lequel une couche perforée ou une couche de contact avec le corps en feuille recouvre le composant absorbant ou un ensemble du composant absorbant et du composant de rembourrage.

9. Pansement selon la revendication 1 qui comprend un agent thérapeutique.

10. Pansement selon la revendication 1 dans lequel le composant de rembourrage est en contact direct avec le composant absorbant.

11. Procédé de fabrication d'un pansement selon la revendication 1, lequel procédé comprend, comme première étape, le recouvrement au moins partiellement de la face distale d'un précurseur de composant absorbant avec la face proximale d'un précurseur de composant de rembourrage.
